# EUROPÄISCHE PATENTSCHRIFT

(11) **EP 3 413 907 B1**
(45) Veröffentlichungstag und Bekanntmachung des Hinweises auf die Patenterteilung: **13.01.2021**
(21) Anmeldenummer: 17704004.5
(22) Anmeldetag: 09.02.2017
(51) Int. Cl.: A61K 38/16, A61M 1/34, A61M 1/36

(54) **BLUTBEHANDLUNG MIT INAKTIVIERUNG VON FREIEN NUKLEINSÄUREN**
BLOOD TREATMENT WITH INACTIVATION OF CIRCULATING NUCLEIC ACIDS
TRAITEMENT DU SANG PAR INACTIVATION D'ACIDES NUCLÉIQUES LIBRES

(30) Priorität: 09.02.2016 DE 102016001407
(43) Veröffentlichungstag der Anmeldung: 19.12.2018
(73) Patentinhaber: Fresenius Medical Care Deutschland GmbH, 61352 Bad Homburg (DE)
(72) Erfinder: LAUBROCK, Andreas, 61273 Wehrheim (DE); FISLAGE, Rainer, 66606 St. Wendel (DE)
(74) Vertreter: Stolmár & Partner Patentanwälte PartG mbB
(86) Internationale Anmeldenummer: PCT/EP2017/052871
(87) Internationale Veröffentlichungsnummer: WO 2017/137495

(56) Entgegenhaltungen:
- EP-A1- 1 666 055
- WO-A1-2005/025650
- US-A- 4 824 432
- US-A1- 2011 125 286

## Beschreibung

Die vorliegende Erfindung bezieht sich auf eine Vorrichtung zur Blutbehandlung, umfassend eine feste Phase, auf der Polypeptide zur Inaktivierung von freien Nukleinsäuren immobilisiert sind, und einen Dialysator oder einen Hämofilter, wobei die feste Phase dem Dialysator oder Hämofilter vorgeschaltet ist oder sich im Dialysator oder Hämofilter befindet.

Patienten, die mit Verfahren der Hämodialyse oder Peritonealdialyse behandelt werden, weisen im Vergleich zur Normalbevölkerung erhöhte Spiegel von proinflammatorischen Cytokinen wie z.B. TNF-α, IL-6, IL-12 und IL-1β auf, die über den NFκB-Signalweg ausgeschüttet werden. Bezüglich der zugrunde liegenden Ursache gibt es unterschiedliche Erklärungsansätze. In jedem Fall sind diese erhöhten Konzentration an Entzündungsstoffen mit schlechtem Überleben der Patienten bei chronischer Dialyse gekoppelt (Panichi V, Paoletti S et al. Inflammatory pattern in hemodiafiltration. Contrib. Nephrol. 2008; 161: 185-190), so dass der Bedarf besteht, die Induktion der zugrunde liegenden Signalkaskaden ursächlich zu unterbinden. Kann man die Freisetzung der proinflammatorischen Cytokine unterbinden, so erwächst den Patienten ein Überlebensvorteil.

Eine von mehreren bekannten Ursachen für die Induktion und Freisetzung von proinflammatorischen Cytokinen ist der Kontakt von frei im Blut zirkulierender DNA mit Rezeptoren, die PAMP-Strukturen (pathogen associated molecular patterns) erkennen. Die zugehörigen PRR-Proteine (pattern recognition receptors), die die PAMP detektieren, sind ein phylogenetisch altes, heterogenes System von Rezeptormolekülen, das grundlegende Strukturen von Krankheitserregern erkennt und entsprechende Signale zur Aktivierung des Immunsystems aussendet.

So induziert beispielsweise von gramnegativen Bakterien freigesetztes Lipopolysaccharid (LPS, Synonym: Endotoxin) bei Kontakt mit Vollblut bereits in geringsten Mengen die Freisetzung von proinflammatorischen Cytokinen. Die Erkennung des biologisch wirksamen Lipid A-Anteils des Endotoxins erfolgt über den TLR-4-Rezeptor (toll-like receptor), der in der Lage ist, die Lipid A-Strukturen von verschiedenen Bakterienspezies zu erkennen, obwohl sich diese im Feinaufbau unterscheiden (Medzhitov RM, Janeway C. Innate Immunity. N. Engl. J. Med. 2000; 338: 338-344).

Weiterhin werden auf Bakterienzellen vorhandene Phosphorylcholingruppen durch das C-reaktive Protein des menschlichen Blutes erkannt. Durch nachgeschaltete komplementvermittelte Aktivierungsvorgänge werden auch in diesem Fall proinflammatorische Cytokine ausgeschüttet (Tillet, WS, Francis TJ. Serological reactions in pneumonia with a non-protein somatic fraction of pneumococcus. J. Exp. Med. 1930; 52: 561-585. Black S, Kushner I et al. C-reactive protein. J. Biol. Chem. 2004; 279: 48487-48490).

Für die Erkennung von DNA wurde ebenfalls ein PRR beschrieben, das den Namen TLR-9 trägt (Chi H, Flavell RA. Innate recognition of non-self nucleid acids. Genome Biol. 2008; 9:211). Bisher wurde gezeigt, dass TLR-9 bakterielle DNA erkennt und durch diese aktiviert wird. Eine Reaktion von TLR-9 auf endogene DNA des menschlichen Körpers war jedoch noch nicht bekannt, weswegen in Fachkreisen die Kontamination der Dialysierflüssigkeit mit bakteriellen DNA-Fragmenten diskutiert wurde (Kwan BCH, Chow KM et al. Effect of using ultrapure dialysate for hemodialysis on the level of circulating bacterial fragment in renal failure patients. Nephron. Clin. Pract. 2013; 123: 246-253. Schindler, R, Beck W et al. Short bacterial DNA fragments: detection in the dialysate and induction of cytokines. J. Am. Soc. Nephrol. 2004; 15: 3207-3214).

Jedoch war auch bekannt, dass im Blut von CKD-Patienten erhöhte Mengen an freier apoptotischer DNA vorliegen. Die genaue Herkunft der DNA sowie deren Freisetzungsmechanismus sind noch nicht ergründet. Da auch im Blut von Normalpersonen freie zirkulierende Nukleinsäuren nachweisbar sind (Tamkovich, SN, Bryzgunova, OE et al. Circulating nucleic acids in blood of healthy male and female donors. Clin. Chem. 2005; 7: 1317-1319), besteht entweder die Möglichkeit, dass bei CKD-Patienten erhöhte Bildungsraten aufgrund von vermehrter Apoptose vorliegen, oder aber, dass der Abbau der DNA hier inhibiert ist. Insbesondere durch die urämische Stoffwechsellage der Patienten entstehen möglicherweise Substanzen, die die Aktivität von Desoxyribonukleasen, also genau den Enzymen, die für den Abbau von DNA sorgen, inhibieren. Kürzlich konnte nun gezeigt werden, dass diese freie DNA für die Freisetzung von proinflammatorischen Cytokinen verantwortlich ist (Atamaniuk J, Kopechky C et al. Apoptotic cell-free DNA promotes inflammation in haemodialysis patients. Nephrol. Dial. Transplant. 2011; 0:1-4).

Im Bereich der Herz-Kreislauf-Forschung finden sich ebenfalls Hinweise auf den Ursprung der freien DNA. Hier wurde nachgewiesen, dass nicht die genomische menschliche DNA aus apoptotischen Abbauvorgängen für die Cytokininduktion verantwortlich ist, sondern intrazellulär lokalisierte mitochondriale DNA (mtDNA) (Oka T, Hikoso S et al. Mitochondrial DNA that escapes from autophagy causes inflammation and heart failure. Nature. 2012; 485: 251-255). Analog bedeutet das, dass im Blut von CKD-Patienten nicht nur bakterielle DNA-Fragmente für die Cytokininduktion verantwortlich sind, sondern vor allem menschliche mitochondriale DNA, die mindestens teilweise in freier Form im Blut vorliegt. Aus phylogenetischen Gründen (Symbiontentheorie) weist mitochondriale DNA, ebenso wie bakterielle DNA, ein statistisch unterrepräsentiertes CpG-Dinukleotid auf, das zudem - im Gegensatz zur genomischen DNA - nur in geringem Maße methyliert ist. Aufgrund dieser Ähnlichkeit mit einem PAMP (nicht-methylierte CpG-Dinukleotide) kann somit frei zirkulierende mitochondriale DNA den TLR-9-Rezeptor aktivieren, der dann seinerseits die Cytokinsynthese induziert.

Frei zirkulierende DNA spielt auch eine Rolle beim Krankheitsbild des Lupus erythematodes (LE), einer genetisch bedingten Autoimmunerkrankung. So wurden im Blut von LE-Patienten freie Nukleinsäuren nachgewiesen, die die Bildung von Anti-DNA-Antikörpern induzieren und somit Cytokinausschüttung und Entzündungsreaktionen hervorrufen (Tan EM, Schur PH et al. Deoxyribonucleic acid (DNA) and antibodies to DNA in the serum of patients with systemic lupus erythematosus. J. Clin. Invest. 1966; 46: 1732-1740). Diese Anti-DNA-Antikörper tragen auch erheblich zu renalen Symptomen bei LE-Patienten bei (Termaat, RM, Asmann, M et al. Anti-DNA antibodies can bind to the glomerulus via two distinct mechanisms. Anti-DNA antibodies. 1992: 61).

In Krebspatienten wurden erhöhte Konzentrationen einer anderen Art von freien Nukleinsäuren beschrieben, nämlich von mRNAs (Tani N, Ichikawa D et al. Circulating Cell-free mRNA in Plasma as a Tumor Marker for Patients with Primary and Recurrent Gastric Cancer. Anticancer Research. 2007; 27: 1207-1212. Garcia V, Garcia, JM et al. Free circulating mRNA in plasma from breast cancer patients and clinical outcome. Cancer Letters. 2008; 263: 312-320. March-Villaalba JA, Martinez-Jabaloyas JM et al. Cell-Free Circulating Plasma hTERT mRNA Is a Useful Marker for Prostate Cancer Diagnosis and Is Associated with Poor Prognosis Tumor Characteristics. PLoS One. 2012; 7(8): e43470.) Es ist bekannt, dass Entzündungsreaktionen allgemein zur Tumorprogression beitragen (Coussens LM, Werb Z, Inflammation and cancer. Nature. 2002; 420: 860-867). Neuere Studien legen zudem nahe, dass auch bei Krebs inflammatorische Zustände durch die Aktivierung von PRR wie dem TLR-7-Rezeptor verursacht werden (Chatterjee S, Crozet L et al. TLR7 Promotes Tumor Progression, Chemotherapy Resistance, and Poor Clinical Outcomes in Non-Small Cell Lung Cancer. Cancer Res. 2014; 74: 5008-5018). Als Mediator der entzündlichen Reaktionen kommen auch hier freie Nukleinsäuren wie mRNAs in Frage.

Somit ergibt sich, dass durch Inhibierung der biologischen Wirkung von frei zirkulierenden Nukleinsäuren bei den genannten Krankheitsbildern die Induktion des proinflammatorischen Cytokinsystems verhindert werden kann. Diese Inhibierung lässt sich durch gezielte Inaktivierung der Nukleinsäuren erreichen, zum Beispiel durch direkte Degradation an fester Phase oder Degradation in flüssiger Phase oder durch Methylierung oder Deaminierung der DNA, wodurch das PAMP Motiv unkenntlich gemacht wird und eine Aktivierung von TLR-9 und somit eine Induktion der Cytokinsynthese nicht mehr möglich ist.

Im Stand der Technik wird beschrieben, dass Krebspatienten DNasel, eine Desoxyribonuklease, verabreicht werden kann, um im Blut frei zirkulierende DNA abzubauen (EP 1655036 B1). Dieses Verfahren birgt jedoch zahlreiche Risiken und Probleme, da das entsprechende Enzym direkt in den Körper des Patienten gelangt. Zum einen ist eine relative hohe Dosierung nötig, um überhaupt eine entsprechende Wirkung zu erzielen. Andererseits sind Nebenwirkungen, die zum Beispiel durch Internalisierung des aktiven Enzyms in Zellen verursacht werden, nicht auszuschließen.

In der US 4,82,432 A werden Verfahren und Vorrichtungen zur Behandlung von Krankheiten beschrieben, die mit der erhöhten Produktionen von Interferonen einhergehen, z.B. AIDS. Dabei werden auch "enzyme disintegration methods" erwähnt. Jedoch offenbart die D1 keine Vorrichtung umfassend einen Dialysator oder Hämofilter.

US 2011/0125286 A1 beschreibt eine künstliche Leber, in welcher Leberzellen mit mechanischen Blutreinigungsvorrichtungen kombiniert werden. Diese kann auch ein DNA-Entfernungsmodul umfassen. Allerdings offenbart US 2011/0125286 A1 keinen Dialysator oder Hämofilter.

EP 1 666 055 A1 beschreibt Behandlungsverfahren von onkologischen und infektiösen Krankheiten, die auf der Anwendung von DNase I zum Abbau freier zirkulierender DNA beruhen. Hierbei wird die DNasel jedoch nicht mit einer extrakorporalen Blutbehandlung kombiniert.

Aufgabe der vorliegenden Erfindung ist es somit, Vorrichtungen und Verfahren bereitzustellen, um frei zirkulierende Nukleinsäuren im Blut auf verbesserte Art und Weise zu inaktivieren.

Die Erfindung löst diese Aufgabe, indem eine Vorrichtung zur Blutbehandlung bereitgestellt wird, enthaltend
(a) Leitungen für das Durchfließen von Vollblut oder Plasma von und zum Patienten und
(b) eine feste Phase, auf der ein Polypeptid immobilisiert ist, welches zur Inaktivierung von freien Nukleinsäuren geeignet ist, und
(c) einen Dialysator oder Hämofilter,
wobei die feste Phase dem Dialysator oder Hämofilter vorgeschaltet ist oder sich im Dialysator oder Hämofilter befindet..

Die feste Phase der erfindungsgemäßen Vorrichtung, auf der das Polypeptid immobilisiert ist, kann sowohl eine Hohlfasermembran als auch Beads oder ein Faservlies sein. Im Rahmen der vorliegenden Erfindung kann die Membran aus para-Methoxymethamphetamin (PMMA), Cellulose, Ester, Polyamid, Polymethylsulfonat, Polyetherimid, Polyethersulfon oder Polysulfon allein oder im Gemisch mit Polyethylenimin, Polyvinylpyrrolidon (PVP) oder Copolymereren bestehen. Weiterhin kann die Membran zusätzliche Modifikationen tragen oder unmodifiziert sein. Die Anzahl der Lagen der Membran ist nicht beschränkt; besonders bevorzugt ist eine Ausführungsform mit einer Hohlfasermembran mit drei Lagen.

Die Immobilisierung des Polypeptids kann auf verschiedene Arten, die dem Stand der Technik nach bekannt sind, erfolgen. Für den Erhalt optimaler enzymatischer Aktivität bei Festphasen-immobilisierten Enzymen ist in der Regel ein Linker mit optimierter Länge erforderlich. Die optimale Länge dieses Abstandshalters ist von der Art der Oberfläche und der Art des Enzyms abhängig und kann leicht vom Fachmann bestimmt werden.

Eine erfindungsgemäße feste Phase kann bevorzugt durch Verspinnen eines polymergebundenen Polypeptids erhalten werden.

Weiterhin kann die offenbarte feste Phase durch Immobilisierung des Polypeptids auf der Oberfläche von Hohlfasermembranen erhalten werden. Hierfür kommen Verfahren in Frage, die auf dem Vorhandensein aktivierter, aktivierbarer oder reaktiver chemischer Funktionen auf der Innenfläche der Membran basieren. Als aktivierbare Gruppen kommen dabei u.a. OH-, COOH-Gruppen in Frage. NH₂- oder SH₂-Gruppen auf einer Oberfläche können auch mit einer chemisch aktivierten Gruppe eines Liganden umgesetzt werden.

Ein Beispiel gibt es in der US 4,177,038. Neben der dort beschriebenen Methode, die beispielsweise bei Cellulose-basierten Membranen zur Anwendung kommen kann, besteht auch die Möglichkeit, Carboxyl-modifizierte Polysulfone oder Polyethersulfone allein oder nicht-modifizierte Polysulfone in Kombination mit Carboxyl-modifizierten PVP-Derivaten einzusetzen.

Beispielsweise kann eine offenbarte feste Phase erhalten werden durch:
- Verspinnen eines Carboxyl-haltigen Polymers auf Basis von Polysulfon oder PVP
- Herstellung von festen Phasen aus den so erzeugten Faserbündeln
- Konditionieren der festen Phasen mit Dampfstoß, Spülung und Trocknung
- Aktivieren der Carboxylgruppen mit einer Lösung aus EDC und ggf. N-Hydroxysuccinimid
- Einführen eines Linkers mit einer Länge von z.B. 6 Kohlenstoffatomen (Aminocapronsäure)
- Erneutes Aktivieren der endständigen Carboxylgruppe mit EDC und anschließender Umsatz mit einer Polypeptidlösung zur Immobilisierung des Polypeptids - dabei werden Aminogruppen (Lysine) des Polypeptids für die kovalente Bindung an die aktivierte Carboxylgruppe des Polymers verwendet
- Spülen der festen Phase und schonendes Trocknen
- Sterilisation der festen Phase mit Gammastrahlung oder vorzugsweise dem EBeam-Verfahren.

Der Begriff "Inaktivieren von freien Nukleinsäuren" umfasst im Rahmen der vorliegenden Erfindung alle enzymatischen Aktivitäten, die geeignet sind, die biologische Aktivität von freien Nukleinsäuren zu inhibieren. Dazu zählt in einem Aspekt der Erfindung das Abbauen oder Degradieren der freien Nukleinsäuren durch Spaltung der Phosphodiester-Bindung zwischen den Nukleotiden der freien Nukleinsäuren. In einem anderen Aspekt umfasst Inaktivieren die Unkenntlichmachung von immunogenen Motiven der freien Nukleinsäuren, zum Beispiel durch Methylierung unmethylierter CpG-Dinukleotide oder Umwandlung eines Cytosins in Uracil durch Deaminierung.

Da die Inaktivierung der freien Nukleinsäuren extrakorporal erfolgt, sind Nebenwirkungen durch Aktivität des Polypeptids im Körper ausgeschlossen. Zudem kann die Vorrichtung mehrfach verwendet werden, was die Therapie besonders kostengünstig im Vergleich mit der direkten Verabreichung des entsprechenden Polypeptids macht.

Der Begriff "freie Nukleinsäuren" umfasst im Rahmen der Erfindung alle Arten von zellfreien Nukleinsäuren, die im Blut zirkulieren. Die Herkunft der freien Nukleinsäuren ist dabei nicht beschränkt. Es ist denkbar, dass die freien Nukleinsäuren aus apoptotischen oder nekrotischen Zellen stammen oder aber von intakten Zellen sezerniert werden. Freie Nukleinsäuren können sowohl aus menschlicher genomischer oder mitochondrialer DNA als auch aus mRNA bestehen. Ebenfalls umfasst sind virale sowie bakterielle DNA, einzel- und doppelsträngige RNA sowie Fragmente davon, die zum Beispiel im Rahmen einer Infektion in den Blutkreislauf des Patienten gelangt sind. Die freien Nukleinsäuren, die durch die vorliegende Erfindung inaktiviert werden, können sowohl an Proteine gebunden sein als auch durch sie stabilisiert werden. Die Länge der freien Nukleinsäuren ist nicht beschränkt und reicht von 20 Nukleotiden/Basenpaaren bis zu mehreren Millionen Basenpaaren. In einer Ausführungsform sind die zu inaktivierenden freien Nukleinsäuren inflammatorisch wirksam.

Erfindungsgemäß wird die biologische Aktivität der freien Nukleinsäuren durch enzymatische Inaktivierung an der festen Phase unterbunden. Dazu werden geeignete Polypeptide eingesetzt. In einer Ausführungsform sind an der festen Phase Desoxyribonukleasen und/oder Ribonukleasen immobilisiert. Diese Enzymklassen umfassen sowohl Endonukleasen als auch Exonukleasen. In einer bevorzugten Ausführungsform wird die humanidentische Endonuklease DNasel verwendet, welche unter dem Handelsnamen "Pulmozyme" zum Beispiel zum Abbau hochmolekularer DNA in der Lunge von Muskoviszidose-Patienten eingesetzt wird. Erfindungsgemäß können auch unterschiedliche Arten von Polypeptiden mit Nuklease-Aktivität gleichzeitig immobilisiert werden. In einer bevorzugten Ausführungsform kann zum Beispiel DNasel mit einer Exonuklease gemeinsam verwendet werden, was zu einer deutlich stärkeren Reduktion des Molekulargewichts der DNA und zu einer besseren Dialysierbarkeit der Bruchstücke der freien Nukleinsäuren führt.

In einer weiteren Ausführungsform handelt es sich bei dem immobilisierten Polypeptid um ein Enzym, dessen Aktivität die Unkenntlichmachung des Motivs von unmethylierten CpG-Dinukleotiden zur Folge hat, wie z.B. Cytosin-Deaminasen und DNA-Methyltransferasen. Ein bevorzugtes Beispiel für eine DNA-Methyltransferase ist humane DNMT1.

Im Rahmen der vorliegenden Erfindung ist die Anwendung von Enzymen und Polypeptiden, die in ihrer Proteinsequenz mit der des Menschen übereinstimmen, bevorzugt, da so die Bildung von Antikörpern bei mehrfacher Anwendung unterbunden werden kann. Weiterhin wird in Betracht gezogen, Polypeptide zu verwenden, die z.B. eine größere Hitze- oder Lösemittelstabilität oder andere verbesserte Eigenschaften aufweisen. Der Fachmann weiß, wie derartige Mutanten über gezielte Mutagenese generiert und auf ihre Eigenschaften hin untersucht werden können.

Erfindungsgemäß umfasst der Begriff "Polypeptide" neben den Enzymen mit der vollen Sequenz auch verkürzte und veränderte Varianten der genannten Enzyme, die die gewünschte Funktionalität zeigen. Die Varianten weisen hierbei eine Sequenzhomologie von 60, 65, 70, 75, 80, 85, 90, 92, 95 oder 100% auf. Bevorzugt haben die Varianten eine Sequenzhomologie von 75, 80, 85, 90, 92, 95 oder 100%. Auch können nur einzelne Domänen der entsprechenden Enzyme verwendet werden, wenn diese die benötigte katalytische Aktivität aufweisen. Methoden, um die katalytische (Rest-)Aktivität eines Polypeptids zu messen, sind dem Fachmann hinreichend bekannt und umfassen funktionale Assays, Aktivitätsassays und Assays mit chemoluminiszenten oder fluoreszierenden Substraten oder Substraten, deren Umsetzung spektrophotometrische Änderungen hervorruft.

In einer weiteren Ausführungsform werden kurzkettige Peptidsequenzen mit gewünschter enzymatischer Aktivität verwendet. Peptide mit Nuklease-Aktivität werden zum Beispiel in der WO02/40631 A2 beschrieben. Derartige Peptide haben den Vorteil, einem technischen Sterilisationsprozess zu widerstehen. Auch von diesen Peptiden kann der Fachmann die entsprechende enzymatische Aktivität mit bekannten Mitteln messen.

Die Leitungen der erfindungsgemäßen Vorrichtung können mit einem Patienten direkt verbunden sein. Alternativ können weitere Leitungen bereitgestellt werden, über die der Patient mit der Vorrichtung in Flüssigkeitsverbindung steht. Die Leitungen dienen dem Durchfließen des Vollbluts bzw. Plasmas.

Die erfindungsgemäße Vorrichtung enthält weiterhin einen Dialysator oder Hämofilter. Verfahren zur Dialyse und Hämofiltration sind dem Fachmann bekannt und bezeichnen in diesem Zusammenhang alle Methoden, die unter Verwendung von Trennmembranen unerwünschte Substanzen aus den Körperflüssigkeiten von Patienten, die von einer fehlenden oder reduzierten renalen Ausscheidungsfunktion betroffen sind, eliminieren.

Dazu gehört das Verfahren der Hämodialyse, bei dem die unerwünschten Substanzen aus dem Blut entfernt werden, indem das Blut mit einer Dialysierflüssigkeit in Kontakt gebracht wird. Als Trenngrenze zwischen Blut und Dialysierflüssigkeit werden Polymermembranen eingesetzt, die einen diffusiven und konvektiven Stoffaustausch erlauben.

Ebenso kann das Verfahren der Peritonealdialyse eingesetzt werden. Hier dient das Peritoneum des Patienten als Trenngrenze für den Stoffaustausch. Das Bauchfell trennt in diesem Fall die in den Bauchraum eingebrachte Dialysierflüssigkeit von den Körperflüssigkeiten des Patienten. Der Stoffaustausch erfolgt im Wesentlichen diffusiv.

Für die genannten Dialyseverfahren gibt es eine Vielzahl technischer Variationen, die dem beschriebenen Grundprinzip folgen. Aus physiologischen Gründen ist allen Verfahren gemeinsam, dass keine Substanzen, deren Molekulargewicht oberhalb von etwa 69 kDa, dem Molekulargewicht des Albumins liegen, dauerhaft aus dem Körper des Patienten entfernt werden. Bedingt durch das hohe Molekulargewicht doppelsträngiger DNA von etwa 650 Da/Basenpaar (Bp) können bereits kurze DNA-Fragmente im Bereich von 100 bp durch eine Dialysemembran mit einer Trenngrenze von 69 kDa nicht mehr effektiv abgetrennt werden.

Neben ortsgebundenen Systemen sind dem Stand der Technik nach auch am Patienten tragbare Systeme bekannt, die unter dem Kürzel "WAK" (wearable artificial kidney) bekannt sind, oder portable Systeme, die zwar ortsfest betrieben werden, aber gut transportabel sind.

Im Rahmen der vorliegenden Erfindung ist die feste Phase mit dem immobilisierten Polypeptid dem Dialysator oder Hämofilter vorgeschaltet oder befindet sich innerhalb des Dialysators oder Hämofilters Im erstgenannten Fall stehen die feste Phase mit dem immobilisierten Polypeptid und der Dialysator oder Hämofilter über Leitungen in Flüssigkeitsverbindung. In jedem Fall können die Bruchstücke der inaktivierten freien Nukleinsäuren während der Dialyse aus dem Vollblut oder Blutplasma entfernt werden.

In einer weiteren Ausführungsform enthält die Vorrichtung zur Blutbehandlung weiterhin einen Plasmafilter. Dabei kann es sich zum Beispiel um einen Plasmafilter handeln, der über einen Druckgradienten im vorderen Bereich das vom Patienten kommende Vollblut filtriert, während im hinteren Bereich das gereinigte Plasma rückfiltriert und somit, nach Passieren des Dialysators, dem Patienten erneut zugeführt wird. Die Vorrichtung zur Filtration von Plasma aus dem Gesamtblut kann auch ein Zellseparator, zum Beispiel eine Zentrifuge, sein. Die Vorrichtung zur Filtration von Plasma dient dazu, die zellulären Bestandteile des Blutes zurückzuhalten, die möglicherweise mit der Inaktivierung der freien Nukleinsäuren interferieren. Zusätzlich kann so eine Schädigung der Blutzellen durch das immobilisierte Polypeptid vermieden werden.

Offenbart ist weiterhin die Verwendung der erfindungsgemäßen Vorrichtung zur Behandlung von proinflammatorischen Zuständen bei Patienten, die an chronischem Nierenversagen, Krebs oder Lupus erythematodes leiden. Durch das Entfernen der freien Nukleinsäuren, die durch das innate Immunsystem erkannt werden, kann bei allen drei Krankheitsbildern die Ausschüttung proinflammatorischer Cytokine inhibiert und so die entzündliche Reaktion beim Patienten abgeschwächt werden.

Weiterhin offenbart ist ein Verfahren zur Blutbehandlung, wobei freie Nukleinsäuren extrakorporal inaktiviert werden. Das erfindungsgemäße Verfahren kann in einer Ausführungsform folgende Schritte umfassen:
(i) Bereitstellen einer erfindungsgemäßen Vorrichtung; und
(ii) Durchleiten des Vollbluts oder Blutplasmas eines Patienten durch die Vorrichtung unter Bedingungen, die das Inaktivieren der freien Nukleinsäuren durch das auf der festen Phase immobilisierte Polypeptid ermöglichen.

Insbesondere ist hierbei auf eine angemessene Fließgeschwindigkeit zu achten, auf eine Betriebstemperatur, die eine optimale Enzymaktivität erlaubt, und auf eine ausreichend hohe Dichte an immobilisiertem Polypeptid. Erfindungsgemäß kann der Schritt (ii) des Verfahrens wiederholt werden, um eine bessere Inaktivierung der freien Nukleinsäuren zu erreichen.

In einer weiteren Ausführungsform kann das erfindungsgemäße Verfahren zusätzlich einen Schritt (ia) des Filtrierens des Blutplasmas aus dem Vollblut umfassen.

Dadurch, dass im erfindungsgemäßen Verfahren in der bevorzugten Ausführungsform Dialyse und Inaktivierung der freien Nukleinsäuren kombiniert werden, können bei der Blutbehandlung gleichzeitig die Bruchstücke der freien Nukleinsäuren, die durch die Aktivität des Polypeptids entstehen, aus dem Blutplasma oder Vollblut herausgefiltert werden.

Die Erfindung umfasst weiterhin ein System zur Blutbehandlung, umfassend einen extrakorporalen Kreislauf, der eine erfindungsgemäße Vorrichtung enthält. Das System kann zusätzliche Elemente wie Körperflüssigkeitsfördereinrichtungen, Pumpen, Filter, Adsorber, Steuerelemente, Mess- und Eingabeeinheiten und Datenleitungen enthalten. Es wird in Betracht gezogen, dass das System aus mehreren Flüssigkeitskreisläufen besteht.

In einer bevorzugten Ausführungsform wird das erfindungsgemäße System zur Behandlung von proinflammatorischen Zuständen bei Patienten verwendet, die an chronischem Nierenversagen, Krebs oder Lupus erythematodes leiden.

Die Erfindung ist weiter anhand von Figuren und Beispielen näher erläutert, ohne dass diese als beschränkend verstanden werden sollen. Es zeigt:
Figur 1: ein schematisches Blockschaltbild einer Ausführungsform einer nicht erfindungsgemäßen Vorrichtung, bei der das Polypeptid auf Beadmaterial oder Faservlies auf einer festen Phase immobilisiert ist, ohne zusätzliche Schaltung eines Dialysators und ohne Plasmaseparation.
Figur 2: ein schematisches Blockschaltbild einer Ausführungsform einer erfindungsgemäßen Vorrichtung, bei der das Polypeptid auf Beadmaterial oder Faservlies auf einer festen Phase außerhalb des Dialysators immobilisiert ist.
Figur 3: ein schematisches Blockschaltbild einer Ausführungsform einer erfindungsgemäßen Vorrichtung mit einer mehrlagigen Hohlfasermembran, bei der das Polypeptid lumenseitig immobilisiert ist.
Figur 4: Schema einer erfindungsgemäßen mehrlagigen Hohlfasermembran, bei der lumenseitig das Polypeptid immobisiliert ist.
Figur 5: ein schematisches Blockschaltbild einer Ausführungsform einer erfindungsgemäßen Vorrichtung, bei der zusätzlich eine Plasmaseperation stattfindet.
Figur 6: Agarosegelbild von der Behandlung chromosomaler menschlicher DNA mit DNasel
Figur 7: Agarosegelbild, welches die Wirksamkeit immobilisierter DNasel auf Fasermaterial zeigt

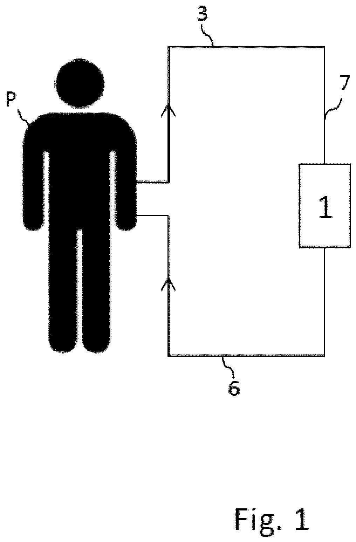

In Figur 1 ist schematisch eine nicht erfindungsgemäße Vorrichtung dargestellt. Die Bezugszeichen dieser Figur sind wie folgt wiedergegeben zugeordnet:
1 Feste Phase, auf der ein Polypeptid immobilisiert ist
3 vom Patienten abführende Leitung
6 zum Patienten zuführende Leitung

Über die Leitung 3 wird das Vollblut oder Blutplasma zur festen Phase 1 geleitet, auf der erfindungsgemäß ein Polypeptid zur Inaktivierung der freien Nukleinsäuren immobilisiert ist. Nach erfolgter Inaktivierung der freien Nukleinsäuen wird das Vollblut oder Blutplasma wieder in den Patienten P zurückgeleitet.

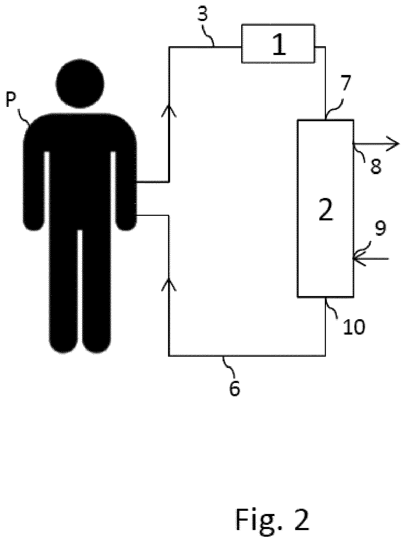

In Figur 2 ist schematisch eine Ausführungsform einer erfindungsgemäßen Vorrichtung dargestellt, bei der Inaktivierung der freien Nukleinsäuren und Dialyse getrennt voneinander stattfinden. Die Bezugszeichen dieser Figur sind wie folgt wiedergegeben zugeordnet:
- 1: Feste Phase in Form von beschichtetem Beadmaterial oder mit beschichtetem Faservlies
- 2: Dialysator
- 3: vom Patienten abführende Leitung
- 6: zum Patienten zuführende Leitung
- 7: Bluteingang
- 8: Dialysatausgang
- 9: Dialysateingang
- 10: Blutausgang
- P: Patient

Bei dieser Aufführungsform wird das Blut zunächst über eine feste Phase 1 in Form von beschichtetem Beadmaterial oder beschichtetem Faservlies geleitet, auf der das Polypeptid immobilisiert ist. Hier werden die im Blut enthaltenen freien Nukleinsäuren degradiert. Nach Passage durch die feste Phase 1 wird das Blut weiter durch den Dialysator 2 geleitet, wo die Bruchstücke der freien Nukleinsäure herausdialysiert werden.

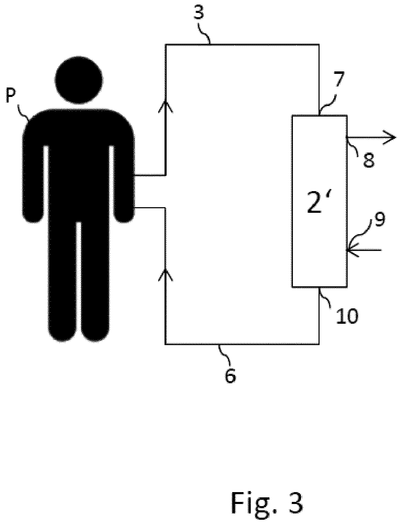

In Figur 3 ist schematisch eine Ausführungsform einer erfindungsgemäßen Vorrichtung dargestellt, bei der das Polypeptid lumenseitig auf einer mehrlagigen Hohlfasermembran innerhalb des Dialysators immobilisiert ist. Die Bezugszeichen dieser Figur sind wie folgt wiedergegeben:
- 2': Dialysator mit lumenseitig immobilisiertem Polypeptid
- 3: vom Patienten abführende Leitung
- 6: zum Patienten zuführende Leitung
- 7: Bluteingang
- 8: Dialysatausgang
- 9: Dialysateingang
- 10: Blutausgang
- P: Patient

Die Leitung 3 weist ein erstes und zweites Ende auf, wobei das erste Ende mit dem Patienten P verbindbar ist und das zweite Ende mit dem Bluteingang 7 des Dialysators 2' verbunden ist. Der Dialysator 2' weist als feste Phase eine mehrlagige Hohlfasermembran auf, auf der das Polypeptid lumenseitig immobilisiert ist. Die Leitung 6 weist ein erstes und ein zweites Ende auf, wobei das erste Ende mit dem Blutausgang 10 des Dialysators 2' verbunden ist und das zweite Ende mit Patienten P verbindbar ist. Das Vollblut, welches durch den Bluteingang 7 eintritt, wird im Dialysator 2' dialysiert, wobei gleichzeitig das immobilisierte Polypeptid die freien Nukleinsäuren inaktiviert; die Bruchstücke werden während des Dialysevorgangs aus dem Blut herausdialysiert. Über den Blutausgang 10 kann das Vollblut wieder austreten und wird über die Leitung 6 wieder dem Patienten zugeführt. Über den Dialysateingang 9 und den Dialysatausgang 8 wird das Dialysat in den Dialysator 2' ein- bzw. ausgeführt.

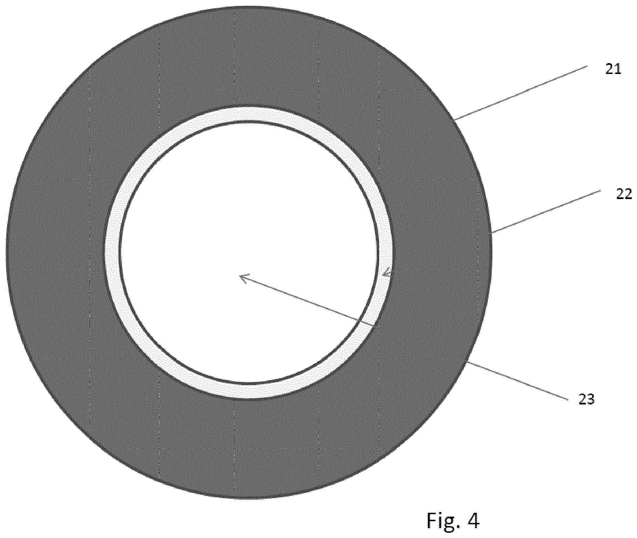

In Figur 4 ist schematisch der Aufbau einer erfindungsgemäßen festen Phase in Form einer mehrlagigen Hohlfasermembran gezeigt, bei der lumenseitig ein Polypeptid immobilisiert ist. Die Bezugszeichen dieser Figur sind wie folgt wiedergegeben zugeordnet:
- 21: äußere Lage des Membranmaterials
- 22: innere Lage des Membranmaterials
- 23: Lumen der Hohlfasermembran

Auf der inneren Lage 22 ist das Polypeptid immobilisiert.

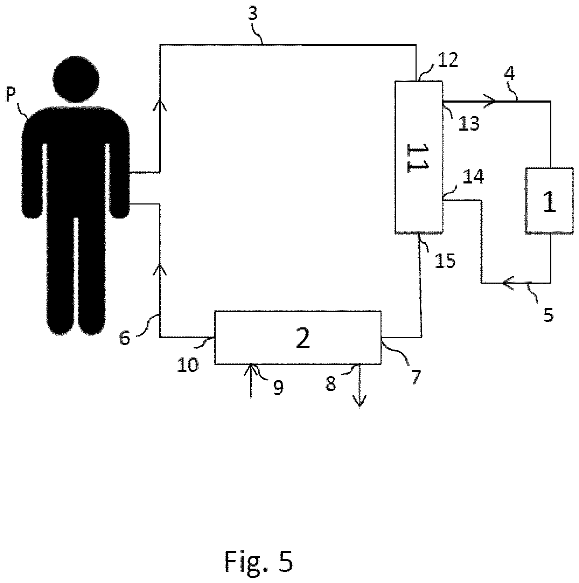

In Figur 5 ist schematisch eine weitere Ausführungsform der erfindungsgemäßen Vorrichtung, umfassend einen Plasmaseparator, dargestellt. Die Bezugszeichen dieser Figur sind wie folgt wiedergegeben zugeordnet:
- 1: Feste Phase, auf der ein Polypeptid immobilisiert ist
- 2: Dialysator
- 3: vom Patienten abführende Leitung
- 4: verbindende Leitung
- 5: verbindende Leitung
- 6: zum Patienten zuführende Leitung
- 7: Bluteingang

- 8: Dialysatausgang
- 9: Dialysateingang
- 10: Blutausgang
- 11: Plasmafilter
- 12: Flüssigkeitszuführeingang der ungefilterten Seite
- 13: Flüssigkeitsabführausgang der ungefilterten Seite
- 14: Flüssigkeitszuführeingang der gefilterten Seite
- 15: Flüssigkeitsabführausgang der gefilterten Seite
- P: Patient

Die Leitung 3 weist ein erstes und zweites Ende auf, wobei das erste Ende mit dem Patienten P verbindbar ist und das zweite Ende mit dem Flüssigkeitszuführeingang 12 einer ungefilterten Seite eines Filters 11 verbunden ist. Der Plasmafilter 11 weist neben der ungefilterten Seite eine gefilterte Seite auf, wobei die ungefilterte Seite von der gefilterten Seite durch zumindest ein Filtermaterial getrennt ist. Die Leitung 6 weist ein erstes und ein zweites Ende auf, wobei das erste Ende mit dem Flüssigkeitsabführausgang 10 des Dialysators 2 verbunden ist und das zweite Ende mit dem Patienten P verbindbar ist. Das Vollblut, welches durch den Flüssigkeitszugang der ungefilterten Seite 12 eintritt, kann als Blutplasma teilweise durch den Flüssigkeitsabführausgang 13 der gefilterten Seite wieder austreten. Über die Leitung 4 wird dann das abgetrennte Blutplasma durch die feste Phase 1, auf der erfindungsgemäß ein Polypeptid immobilisiert ist, geleitet. Das immobilisierte Polypeptid sorgt für die Inaktivierung der im Blutplasma enthaltenen freien Nukleinsäuren. Anschließend fließt das Blutplasma mit den inaktivierten freien Nukleinsäuren über die Leitung 5 und tritt am Flüssigkeitszuführeingang der gefilterten Seite 14 wieder in den Plasmafilter 11 ein und durch den Flüssigkeitsabführausgang 15 der ungefilterten Seite aus. Anschließend wird das Vollblut durch einen Dialysator 2 geleitet, in dem gleichzeitig die Bruchstücke der degradierten freien Nukleinsäuren herausdialysiert werden können. Nach Passage durch den Dialysator 2 wird das gereinigte Vollblut wieder in den Patienten P zurückgeleitet.

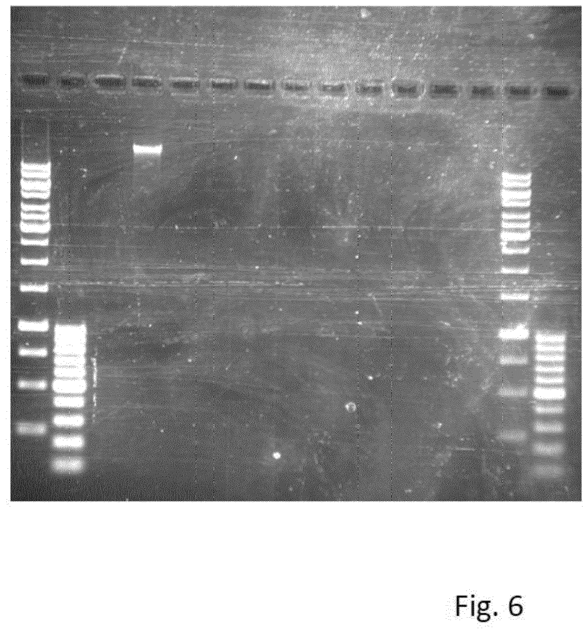

In Figur 6 ist ein Agarosegelbild zu sehen, welches die Behandlung menschlicher chromosomaler DNA mit DNasel zeigt. Die Proben wurden wie folgt aufgetragen (von links nach rechts): 10 µl 1 kB-Leiter, 10 µl 100 bp-Leiter, leer, unbehandelte DNA, 2 U DNasel, leer, 0.5 U DNasel, leer, 0.05 U DNasel, leer, 0.005 U DNasel, leer, 5 µl 1 kB-Leiter, 5 µl 100 bp-Leiter.

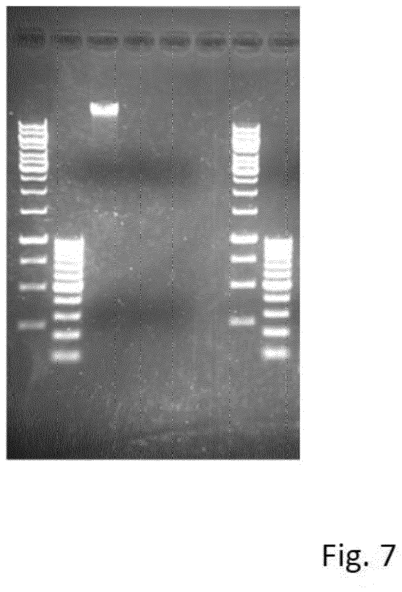

In Figur 7 ist ein Agarosegelbild zu sehen, welches die Wirksamkeit von immobilisierter DNasel zeigt. Die Proben wurden wie folgt aufgetragen (von links nach rechts): 10 µl 1 kB-Leiter, 10 µl 100 bp-Leiter, 400 ng unbehandelte DNA, leer, 400 ng DNA nach Dialysatordurchgang, leer, 5 µl 1 kB-Leiter, 5 µl 100 bp-Leiter.

### Beispiel 1 - Behandlung chromosomaler menschlicher DNA mit DNasel

Hochmolekulare menschliche DNA wurde aus Vollblut nach Lyse der Erythrozyten mit Hilfe der Phenol-Chloroform-Methode gewonnen.

Jeweils 800 ng dieser DNA wurden mit verschiedenen Aktivitäten einer kommerziellen DNAsel-Präparation für 10 min bei 37°C inkubiert. Die Reaktionen wurden nach Ablauf der Reaktionszeit durch Zugabe von Auftragpuffer mit 5 mM EDTA gestoppt und anschließend je 175 ng pro Lane auf ein 1%iges Agarosegel (Tris-Acetat-EDTA-Puffer, 110 V, 2 h) aufgetragen. Als Molekulargewichtsstandard diente eine 1 kB-Leiter mit 10 kB als höchstem Molekulargewicht, eine 100 bp-Leiter sowie 175 ng unverdaute genomische DNA als Referenz.

Nach dem Gellauf wurde mit Ethidiumbromidlösung gefärbt und das Gel unter UV-Beleuchtung per Foto dokumentiert.

**Ergebnis:** Selbst bei einer Konzentration von nur 0.005 U DNAsel werden 800 ng DNA in 10 min bei 37°C vollständig abgebaut, wie am Fehlen des typischen Abbau-Schweifs klar zu erkennen ist. Bei einem Teilabbau der DNA wäre in den betreffenden Lanes eine deutlich erkennbare Anfärbung im Bereich von 10 kbp abwärts zu erkennen gewesen. In der unbehandelten Referenz sind diese Abbauprodukte bereits kurz oberhalb der 10 kBp-Bande zu erkennen (vgl. Figur 6).

### Beispiel 2 - Herstellung von Enzym-beschichteten Hohlfasern

Hohlfasern für die erfindungsgemäße Anwendung der Nukleasen können nach den Methoden aus DE 10 2011 010 921 A1 sowie DE 10 2008 003 090 A1 hergestellt werden. Die dort beschriebenen Membranen enthalten Celluloseester auf ihrer lumenseitigen Schicht, die durch eine etwa 30 min dauernde Behandlung mit verdünnter Natronlauge vollständig oder partiell in Cellulose umgewandelt werden können. Letztere kann durch chemische Aktivierung für eine Bindung biologischer Moleküle vorbereitet werden. Die dabei verwendeten Techniken sind in der US 4,177,038 beschrieben.

Die zu immobiliserende DNasel wird für die Kopplungsreaktion in 5-fachem Überschuss eingesetzt.

### Beispiel 3 - Wirksamkeit immobilisierter DNasel auf Fasermaterial

Um die Effizienz von DNasel, die auf einer Hohlfasermembran immobilisiert ist, zu testen, wurden 3600 U/m² DNasel auf einem Hohlfaser-Dialysator mit 1,4 m² Lumenfläche immobilisiert. Durch diesen Hohlfaser-Dialysator wurden 500 ml frisches Testblut, welches mit 500 µg humaner DNA versetzt war, bei einem Lumenfluss von 150 ml/min bei 37°C "single pass" gepumpt. Der Faserinnendurchmesser des Hohlfaser-Dialysators betrug 185 µm und die Verweilzeit der Flüssigkeit im Dialysator lag bei 28s. Die Dialysatseite des Dialysators war mit isotonischem Dialysat gefüllt, das physiologische Konzentrationen an mono- und divalenten Kationen, insbesondere Calcium und Magnesium, aufwies.

Für die Analyse auf einem Agarosegel wurde eine Probe nach Durchgang durch den Dialysator entnommen. Das Volumen der Probe wurde so gewählt, dass eine Absolutmenge von 400 ng genomischer DNA auf das Gel aufgetragen wurde. Als Referenz diente die unbehandelte genomische DNA in gleicher Menge.

**Ergebnis:** Nach dem Dialysatordurchgang war die genomische DNA vollständig abgebaut, was klar am Fehlen der typischen Abbauprodukte unterhalb von 10kbp erkennbar ist (vgl. Figur 7).

## Patentansprüche

1. Vorrichtung zur Blutbehandlung, umfassend
(a) Leitungen (3, 4, 5, 6) für das Durchfließen von Vollblut oder Blutplasma von und zum Patienten,
(b) eine feste Phase (1), auf der ein Polypeptid immobilisiert ist, welches zur Inaktivierung von freien Nukleinsäuren geeignet ist, und
(c) einen Dialysator (2; 2') oder Hämofilter,
wobei die feste Phase dem Dialysator oder Hämofilter vorgeschaltet ist oder sich im Dialysator oder Hämofilter befindet.

2. Vorrichtung nach Anspruch 1, wobei das Polypeptid ausgewählt ist aus der Gruppe bestehend aus Desoxyribonukleasen, Ribonukleasen, Endonukleasen, Exonukleasen, Endoribonukleasen, Exoribonukleasen oder Peptiden mit Nuklease-Aktivität.

3. Vorrichtung nach Anspruch 1, wobei das Polypeptid ausgewählt ist aus der Gruppe bestehend aus DNA-Methyltransferasen 1 (DNMT1) oder Peptiden mit Methyltransferase-Aktivität.

4. Vorrichtung nach Anspruch 1, wobei das Polypeptid eine Cytosin-Deaminase-Aktivität aufweist.

5. Vorrichtung nach einem der Ansprüche 1 bis 4, wobei die Vorrichtung weiterhin (d) einen Plasmafilter (11) umfasst.

6. Vorrichtung nach einem der Ansprüche 1 bis 5, wobei die feste Phase, auf der das Polypeptid immobilisiert ist, eine Hohlfasermembran, Beads oder Faservliesvlies umfasst.

7. Vorrichtung nach einem der Ansprüche 1 bis 6, wobei die zu inaktivierenden freien Nukleinsäuren inflammatorisch wirksam sind.

8. Vorrichtung nach einem der Ansprüche 1 bis 7, wobei die zu inaktivierenden freien Nukleinsäuren ausgewählt sind aus der Gruppe bestehend aus humaner genomischer DNA, mitochondrialer DNA (mtDNA) oder mRNA.

9. Vorrichtung nach einem der Ansprüche 1 bis 7, wobei die zu inaktivierenden freien Nukleinsäuren ausgewählt sind aus der Gruppe bestehend aus bakterieller oder viraler DNA oder RNA.

10. Polypeptid, das auf einer festen Phase immobilisiert und zur Inaktivierung von freien Nukleinsäuren geeignet ist, zur Behandlung von proinflammatorischen Zuständen bei einem Patienten, wobei die Behandlung umfasst, die inaktivierten freien Nukleinsäuren mittels einer Vorrichtung zur Blutbehandlung aus dem Vollblut oder Blutplasma zu entfernen, die Vorrichtung einen Dialysator oder Hämofilter umfasst und die feste Phase dem Dialysator oder Hämofilter vorgeschaltet ist oder sich im Dialysator oder Hämofilter befindet.

11. System zur Blutbehandlung, umfassend einen extrakorporalen Kreislauf, enthaltend eine Vorrichtung nach einem der Ansprüche 1 bis 9.

## Claims

1. Device for the treatment of blood, comprising
(a) tubes (3, 4, 5, 6) for whole blood or blood plasma to flow through from and to the patient,
(b) a solid phase (1) on which a polypeptide is immobilized which is suitable for the inactivation of free nucleic acids, and
(c) a dialyzer (2; 2') or haemofilter,
wherein the solid phase is upstream of the dialyzer or haemofilter or is located within the dialyzer or haemofilter.

2. Device according to claim 1, wherein the polypeptide is selected from the group consisting of deoxyribonucleases, ribonucleases, endonucleases, exonucleases, endoribonucleases, exoribonucleases or peptides with nuclease activity.

3. Device according to claim 1, wherein the polypeptide is selected from the group consisting of DNA methyltransferases 1 (DNMT1) or peptides with methyltransferase activity.

4. Device according to claim 1, wherein the polypeptide has a cytosine deaminase activity.

5. Device according to any one of claims 1 to 4, wherein the device furthermore comprises (d) a plasma filter (11).

6. Device according to any one of claims 1 to 5, wherein the solid phase on which the polypeptide is immobilized comprises a hollow fibre membrane, beads or a nonwoven.

7. Device according to any one of claims 1 to 6, wherein the free nucleic acids to be inactivated have an inflammatory effect.

8. Device according to any one of claims 1 to 7, wherein the free nucleic acids to be inactivated are selected from the group consisting of human genomic DNA, mitochondrial DNA (mtDNA) or mRNA.

9. Device according to any one of claims 1 to 7, wherein the free nucleic acids to be inactivated are selected from the group consisting of bacterial or viral DNA or RNA.

10. Polypeptide, which is immobilized on a solid phase which is suitable for the inactivation of free nucleic acids, for treating proinflammatory conditions in a patient, wherein the treatment comprises removing the inactivated free nucleic acids from whole blood or blood plasma by means of a device for the treatment of blood, the device comprises a dialyzer or haemofilter and the solid phase is upstream of the dialyzer or haemofilter or is located within the dialyzer or haemofilter.

11. System for the treatment of blood, comprising an extracorporeal circulation containing a device according to any one of claims 1 to 9.

## Revendications

1. Dispositif de traitement du sang, comprenant
(a) des conduits (3, 4, 5, 6) pour la circulation de sang total ou de plasma sanguin en provenance et à destination du patient,
(b) une phase solide (1), sur laquelle un polypeptide, qui est adapté pour l'inactivation d'acides nucléiques libres, est immobilisé, et
(c) un dialyseur (2 ; 2') ou hémofiltre,
dans lequel la phase solide est située en amont du dialyseur ou hémofiltre ou se trouve dans le dialyseur ou hémofiltre.

2. Dispositif selon la revendication 1, dans lequel le polypeptide est sélectionné dans le groupe constitué des désoxyribonucléases, des ribonucléases, des endonucléases, des exonucléases, des endoribonucléases, des exoribonucléases ou des peptides à activité nucléase.

3. Dispositif selon la revendication 1, dans lequel le polypeptide est sélectionné dans le groupe constitué des ADN méthyltransférases 1 (DNMT1) ou des peptides à activité méthyltransférase.

4. Dispositif selon la revendication 1, dans lequel le polypeptide présente une activité cytosine désaminase.

5. Dispositif selon l'une des revendications 1 à 4, dans lequel le dispositif comprend en outre (d) un plasmafiltre (11).

6. Dispositif selon l'une des revendications 1 à 5, dans lequel la phase solide, sur laquelle le polypeptide est immobilisé, comprend une membrane à fibres creuses, des billes ou une nappe fibreuse.

7. Dispositif selon l'une des revendications 1 à 6, dans lequel les acides nucléiques libres à inactiver ont une action inflammatoire.

8. Dispositif selon l'une des revendications 1 à 7, dans lequel les acides nucléiques libres à inactiver sont sélectionnés dans le groupe constitué de l'ADN génomique humain, de l'ADN mitochondrial (ADNmt) ou de l'ARNm.

9. Dispositif selon l'une des revendications 1 à 7, dans lequel les acides nucléiques libres à inactiver sont sélectionnés dans le groupe constitué de l'ADN ou l'ARN bactérien ou viral.

10. Polypeptide, qui est immobilisé sur une phase solide et adapté pour l'inactivation d'acides nucléiques libres, destiné au traitement d'états pro-inflammatoires chez un patient, le traitement comprenant l'élimination du sang total ou du plasma sanguin des acides nucléiques libres inactivés au moyen d'un dispositif de traitement du sang, le dispositif comprenant un dialyseur ou hémofiltre et la phase solide étant située en amont du dialyseur ou hémofiltre ou se trouvant dans le dialyseur ou hémofiltre.

11. Système de traitement du sang, comprenant un circuit extracorporel, doté d'un dispositif selon l'une des revendications 1 à 9.
